# EUROPEAN PATENT SPECIFICATION

(11) **EP 1 909 672 B1**
(45) Date of publication and mention of the grant of the patent: **02.11.2011**
(21) Application number: 06789465.9
(22) Date of filing: 03.08.2006
(51) Int. Cl.: A61B 17/10

(54) **IMPLANT INTRODUCER**
IMPLANTAT-EINFÜHRVORRICHTUNG
INTRODUCTEUR D'IMPLANT

(30) Priority: 04.08.2005 US 705624 P; 04.08.2005 US 705569 P; 28.12.2005 US 754265 P; 19.04.2006 US 745131 P; 01.06.2006 US 810065 P
(43) Date of publication of application: 16.04.2008
(73) Proprietor: C.R.Bard, Inc., Murray Hill, NJ 07974 (US)
(72) Inventor: MEADE, Noah, Atlanta, GA 30350 (US); EVANS, Doug, Snellville, GA 30039 (US)
(74) Representative: Viering, Jentschura & Partner
(86) International application number: PCT/US2006/030581
(87) International publication number: WO 2007/019374

(56) References cited:
- WO-A2-2004/012579
- US-A- 5 501 692
- US-A- 5 755 728
- US-A1- 2005 131 391
- US-B1- 6 210 416

## Description

### CROSS-REFERENCE TO RELATED APPLICATION

This application is related to copending U.S provisional patent applications entitled "Introducer Needle with Extendable Implant Lasso", filed on December 28, 2005, and accorded serial number 60/754,265, and "Introducer Needle With Sliding Sheath", filed on April 19, 2006, and accorded serial number 60/745,131.

### BACKGROUND

Surgical devices referred to as "introducers" are often used to implant or "introduce" implantable devices within the body. For example, such introducers can be used to position within the pelvis mesh implants intended for treating urinary incontinence or performing prolapse repair.

Positioning an implant within the human body, such as within the pelvis, can be challenging due to the anatomy of the body and the placement of the implant that may be required to treat a given ailment. For instance, the treatment of rectocele, a condition in which the rectum encroaches on the vagina, may require accessing the vaginal vault from a position deep within the pelvis so as to form a passage in which a portion, such as an anchoring arm, of the implant can be placed. Formation of such a passage typically requires a relatively high degree of skill.

Further complicating implantation of a rectocele implant, or other such pelvic implant, is the need to draw the implant into the body and through the formed passage. In present techniques, a needle is passed through a pelvic incision, through the soft tissue of the pelvis, into the vagina down through the vagina, and out the vaginal introitus to enable the implant to be connected to the needle so that the needle may then be withdrawn with the implant in tow to position the implant within the formed passage. Given the configuration and dimensions of the human pelvis and its organs, it can be difficult to navigate a needle through such a tortuous path without causing damage to or otherwise disrupting the tissues of the pelvis, such as the pelvic floor muscles.

US 6,210,416 B1 discloses a device, according to the preamble of claim 1.

### DESCRIPTION OF INVENTION

The invention provides an introducer for introducing an implant into a patient's body according to claim 1. Further embodiments of the invention are described in the dependent claims.

### BRIEF DESCRIPTION OF THE DRAWINGS

The disclosed introducers can be better understood with reference to the following drawings. The components in the drawings are not necessarily to scale.
FIG. 1 illustrates a first embodiment of an implant introducer.
FIG. 2 is a perspective view of the introducer of FIG. 1.
FIGs. 3A and 3B illustrate a distal end of the introducer of FIGs. 1 and 2 depicting extension of an internal snare of the introducer.
FIGs. 4A-4C are side views of the introducer of FIGs. 1 and 2 illustrating extension of a sheath of the introducer.
FIGs. 5A-5K illustrate steps performed in a first embodiment of a method for implanting a pelvic implant within the body.
FIGs. 6A and 6B illustrate steps performed in a second embodiment of a method for implanting a pelvic implant within the body.
FIG. 7 is a perspective view of an alternative embodiment of an introducer.

### DETAILED DESCRIPTION

As described above, it can be difficult to position an implant within the body. That may particularly be the case in relation to positioning a pelvic implant intended for use in treating-incontinence or performing prolapse repair. For example, as described above, a surgeon may need to access a point deep within the pelvis, such as the vaginal vault, with an introducer and connect an implant to the introducer at a point outside of the body to enable the implant to be drawn through a passage formed in the soft tissues of the pelvis by the introducer. It is difficult to perform such a procedure with current introducers given that the introducer must traverse a tortuous path to extend outside of the body from a point deep within the pelvis. In addition, unnecessary damage can be inflicted on the patient in positioning the implant.

Disclosed herein are implant introducers that simplify implantation of an implantable device, such as a pelvic implant. The implant can be drawn through the introducer sheath such that the implant traverses the passage formed by the introducer needle without direct contact with the tissues of the passage, thereby reducing irritation to the soft tissues in which the passage is formed.

In the following, various embodiments of introducers and implantation methods are described in detail. Although specific embodiments are presented, those embodiments are mere example implementations of the disclosed introducers and methods and it is noted that other embodiments are possible. All such embodiments are intended to fall within the scope of this disclosure.

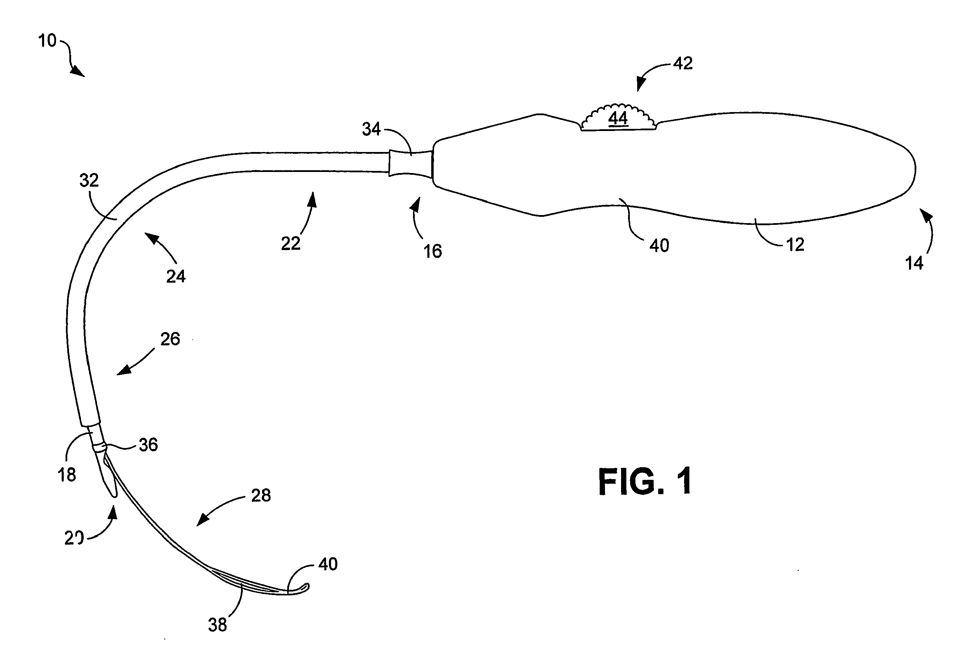

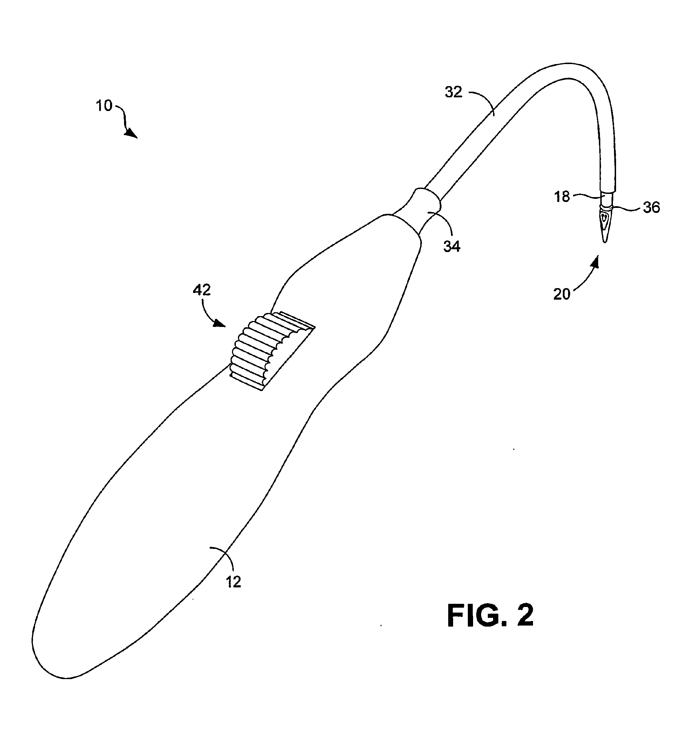

FIGs. 1 and 2 illustrate a first embodiment of an implant introducer 10. The introducer 10 is well suited for use in performing anterior and/or posterior prolapse repair to treat cystocele and rectocele. As indicated in the figures, the introducer 10 comprises a handle 12 that includes a proximal end 14 and a distal end 16. The handle 12 is generally sized and shaped to fit within a surgeon's hand and, as depicted in FIGs. 1 and 2, can be contoured to facilitate firm gripping.

A needle 18 extends from the distal end 16 of the handle 12 and terminates in a blunt point or tip 20 at its distal end that is configured to dissect soft tissue as the needle 22 is passed through the body. In the embodiment of FIGs. 1 and 2, the needle 18 (at least a portion of which is surrounded by a sheath 32; see below) comprises a first generally straight portion 22 adjacent the handle 12, a curved portion 24 in a central region, and a second generally straight portion 26 adjacent its tip 20. The needle 18 is hollow so as to form a cannula in which an extendible internal snare 28 can be positioned. More particularly, the needle 18 forms an inner lumen that extends along substantially the entire length of the needle to one or more openings adjacent the needle tip 20.

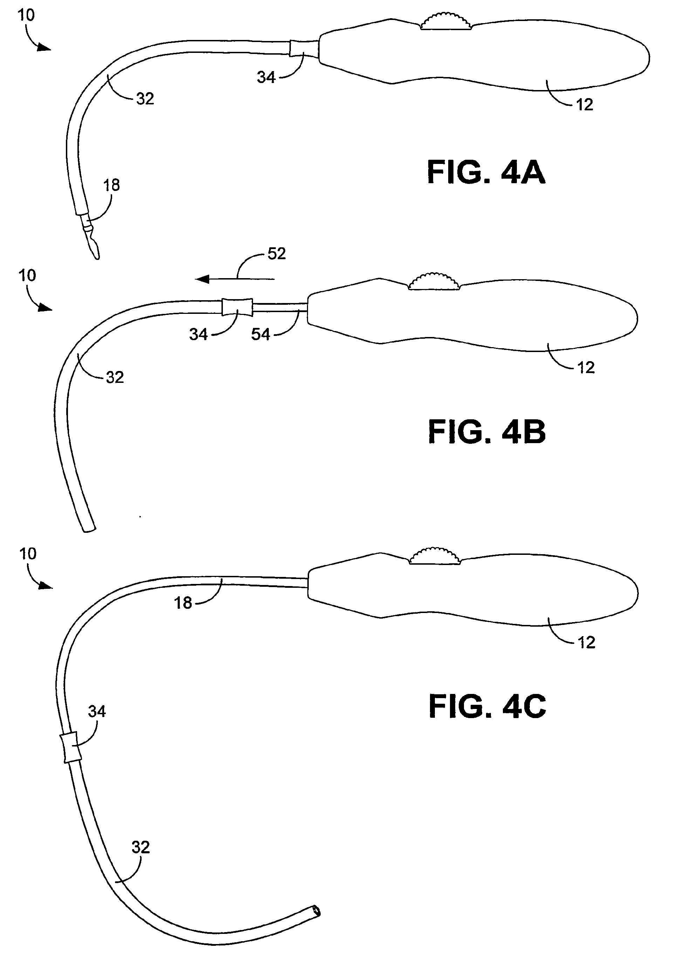

An extendible external sheath 32 surrounds at least part of the needle 18. The sheath 32 is flexible such that it forms to the contours of the needle 18. In the embodiment of FIGs. 1 and 2, the sheath 32, in a "retracted" position shown in FIGs. 1 and 2, extends from the distal end 16 of the handle 12 to a position adjacent the tip 20 of the needle 18. At a proximal end of the sheath 32 is a gripping element 34 that, as described below, is used to change the relative position of sheath and the needle 18. In particular, the sheath 32 can be placed in an "extended" position in which the sheath extends beyond the tip 20 of the needle 18 (see FIG. 4C). Complete removal of the sheath 32 from the needle 18 is prevented due to a stop 36 formed adjacent the needle tip 20 against which the gripping element 34 abuts when the sheath is placed in the extended position.

As is further indicated in FIG.1, the snare 28 forms an implant coupling element 38 adjacent its distal end. As shown, the implant coupling element 38 can comprise a loop through which a portion of an implant can be passed. As described in greater detail below, a constriction 40 can be provided in the implant coupling element 38 to provide a mechanism for securely clamping the implant. Extension and retraction of the snare 28 can be controlled with a extension/retraction mechanism 42 provided on the handle 12. In the embodiment of FIGs. 1 and 2, the mechanism 42 comprises a rotatable element 44, such as a thumb wheel, that can be rotated in a first direction to extend the snare 28, and rotated in the opposite direction to retract the snare.

In terms of materials, the handle 12 can be constructed of any suitable rigid material, such as a metal or a polymeric material. The needle 18 can be constructed of a biocompatible, strong material, such as stainless steel. In some embodiments, the handle 12 and needle 18 can be composed of the same material and may even be unitarily formed together so as to have a monolithic configuration. The sheath 32 may be constructed from flexible biocompatible material. For example, the sheath can comprise a tube of biocompatible polymeric material.

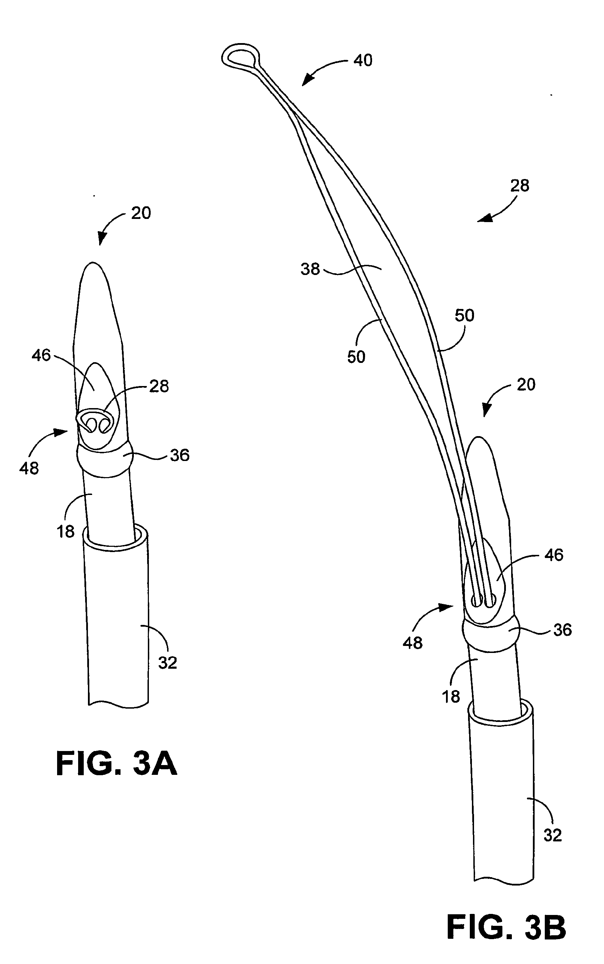

The tip 20 of the needle 18 and the internal snare 28 are depicted in greater detail in FIGs. 3A and 3B. In FIG. 3A, the internal snare 28 is in a fully-retracted position in which only a tip of the snare is outside of the needle inner lumen. In FIG. 3B, the internal snare 28 is in a partially-extended position in which the entire implant coupling element 38 is outside of the needle inner lumen. As shown in both figures, the needle 18 comprises a depression 46 adjacent its tip 20 in which at least one opening 48 is provided that leads to the inner lumen of the needle and through which the snare 28 can pass. In the illustrated embodiment, two such openings 48 are provided, one for each of two legs 50 of the snare 28 (FIG. 3B).

When the share 28 is extended,for example using the mechanism 42 (FIG. 2), the two legs 50 of the snare pass out from the needle 18 through the openings 48 such that the implant coupling element 38 of the snare opens, as indicated in FIG. 3B. The reproducible formation of the loop shape is made possible by forming the legs 50 of the snare 28, and the implant coupling element 38 they form, from a material that has adequate memory to deform to fit within the needle inner lumen, and then spring into its preformed shape when extended out from the lumen. To enable such functionality, the snare 28 can be composed of a polymeric or metal material having shape-memory characteristics. By way of example, the snare 28 is formed from nitinol wire. The implant coupling element 38 provides a space in which a portion of an implant to be introduced into the body can be placed. Once so placed, the implant portion can be urged into the constriction 40 to securely clamp the implant with the snare 28. Notably, although separate "legs" of the snare have been identified, it is to be understood that those legs may merely comprise different portions of the same continuous member (e.g., wire). Furthermore, the implant coupling element 38 can, alternatively, comprise a loop of material that extends from a single wire or shaft.

FIGs. 4A-4C illustrate relative positioning of the introducer needle 28 and its external sheath 32. In the retracted position shown in FIG. 4A, the sheath gripping element 34 is positioned adjacent the handle 12. As shown in FIG. 4B, however, the sheath 32 has been extended relative to the needle 18 and handle 12 by sliding the sheath in the direction indicated by arrow 52. Such sliding can be achieved by the surgeon by holding the introducer 10 handle 12 with one hand and pulling the gripping element 34 in a direction away from the handle. In the extended position shown in FIG. 4C, the sheath 32 has been fully extended to the point at which substantially only the tip 20 of the needle 18 is covered by the sheath. As described above, complete removal of the sheath 32 can be prevented by interaction between the needle stop 36 and the gripping element 34. In particular, internal surfaces of the gripping element 34 can abut the stop 36 before the sheath 32 is removed from the needle 18. Although the sheath 32 has been described as being "extended" from the needle 18, the opposite is also possible. In particular, as described below, the needle 18 can be withdrawn from the sheath 32 to achieve "extension" of the sheath relative to the needle.

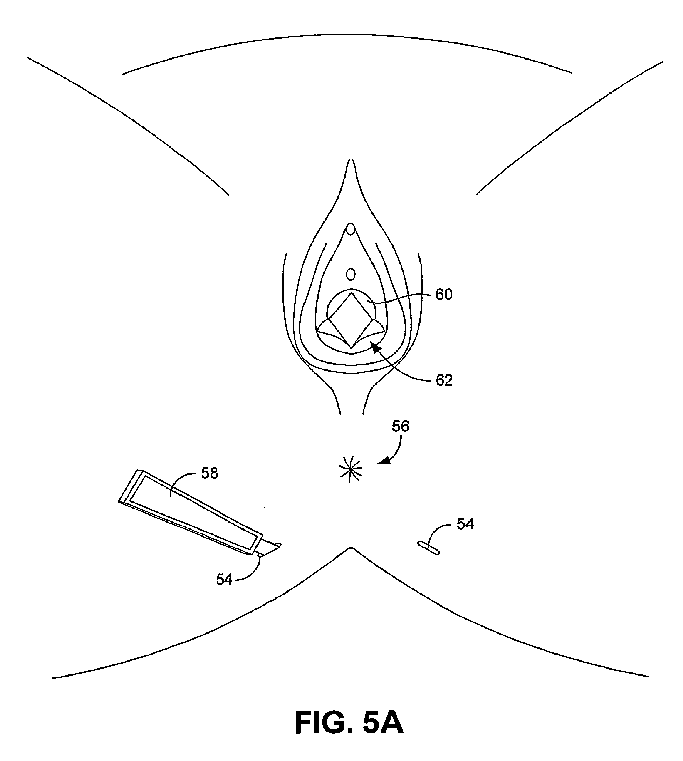

FIGs. 5A-5K illustrate a process for implanting an article using the introducer 10. More particularly, FIGs. 5A-5K illustrate a procedure for implanting a posterior prolapse repair implant between the vagina and the rectum using the introducer 10. Although a posterior repair procedure is depicted in FIGs. 5A-5K and is described in detail in the following for purposes of describing the manner in which the disclosed introducer can be used to introduce an implant, it is to be understood that the procedure is described for purposes of example only. As stated above, similar introducers may be used to implant other implants in other surgical procedures, such as anterior prolapse repair or treatment of urinary incontinence.

Beginning with FIG. 5A, small pararectal incisions 54 are made on either side of the anus 56 with a sharp device, such as a scalpel 58. By way of example, the incisions 56 are made 2-3 centimeters (cm) posterior and lateral to the anus 56. In addition, a midline incision is made in the posterior vaginal wall 60 to form an opening 62 that extends from the vaginal introitus to the vaginal apex to provide access to the space between the vagina and the rectum. The vaginal mucosa may then be dissected away from the rectum using blunt and/or sharp dissection.

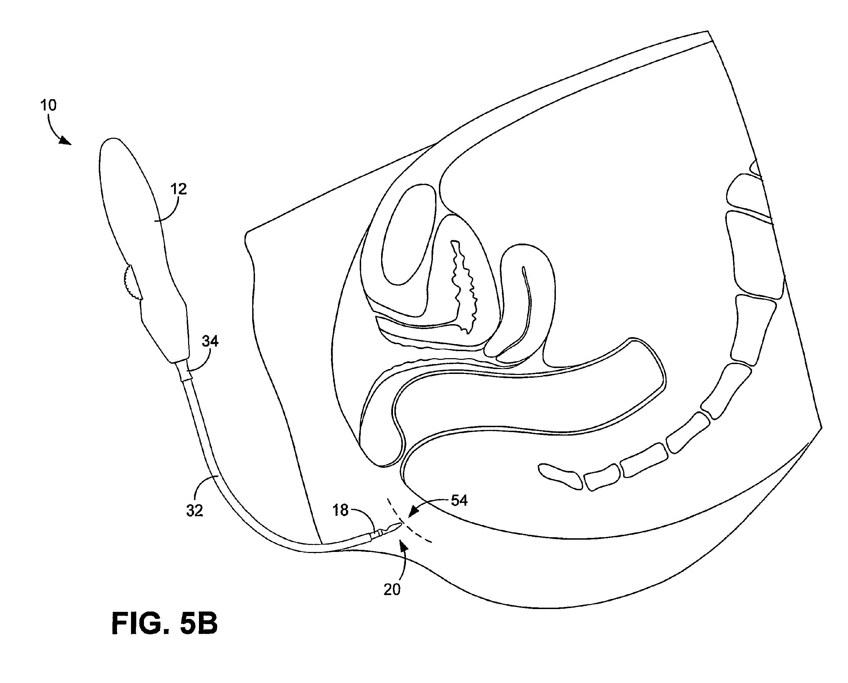

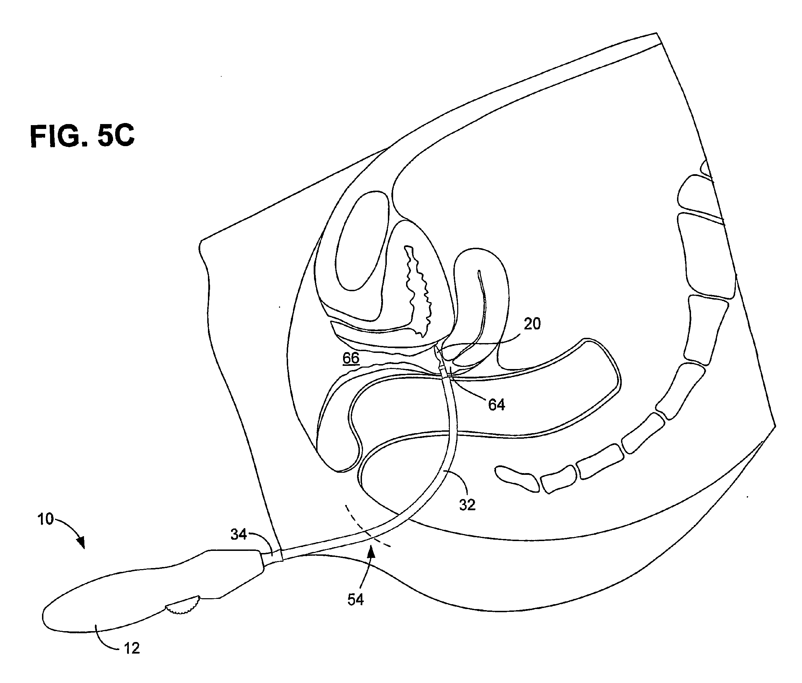

Turning to FIG. 5B, the tip 20 of the introducer needle 18 is positioned at one of the incisions 54 with the introducer 10 oriented so that the handle 12 is generally vertical. Referring next to FIG. 5C, the introducer needle 18 is passed along with its sheath 32 through the incision 54 and through the soft tissue of the pelvis toward the ischial spine (not shown). As the needle 18 and the sheath 32 pass through the soft tissue, the introducer 10 is rotated so that the handle 12 approximates a horizontal orientation, as indicated in FIG. 5C. The needle tip 20 is advanced through the posterior vaginal wall and into the vaginal vault 64 such that the tip is positioned within the vagina 66. That process can be aided by placing a finger within the vagina 66 to guide the needle tip 20 into position.

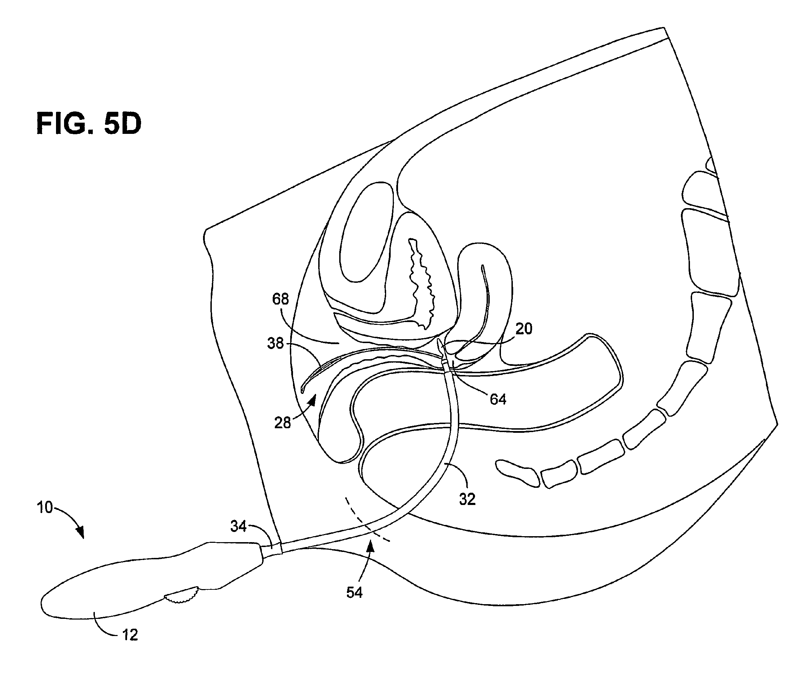

With reference to FIG. 5D, the snare 28 is extended from the retracted position (FIG. 3A) in which the implant coupling element 38 is substantially contained within the inner lumen of introducer needle 18, to an extended position in which the implant coupling element extends to or beyond the vaginal introitus 68, as indicated in FIG. 5D.

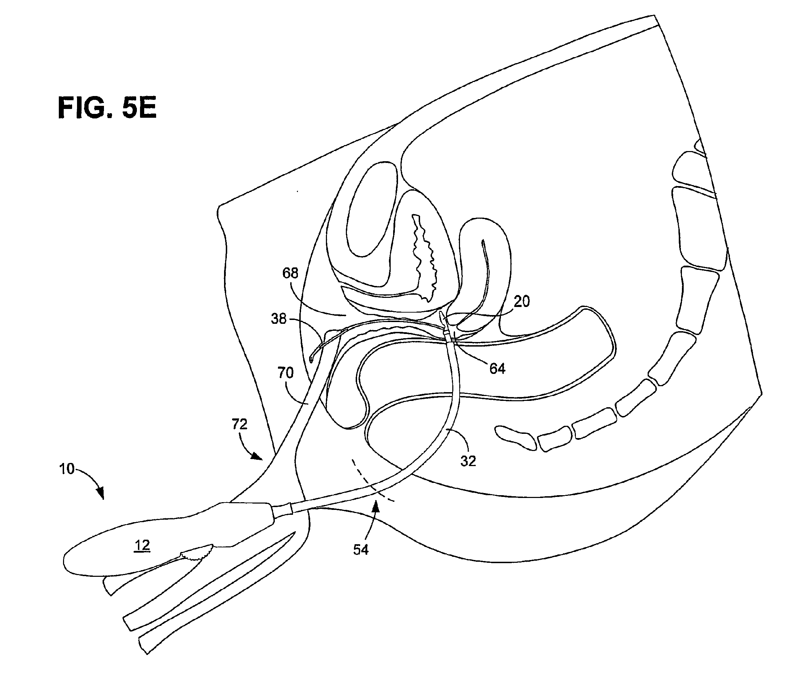

Referring next to FIG. 5E, a relatively long anchoring arm 70 of an implant 72 is coupled to the implant coupling element 38. By way of example, the implant 72 comprises a flexible mesh implant such that the arm 70 can simply be passed through the loop of the coupling element 38. The arm 70 can then, optionally, be urged into the constriction 40 (FIG. 3B) to securely clamp the implant to the snare 28.

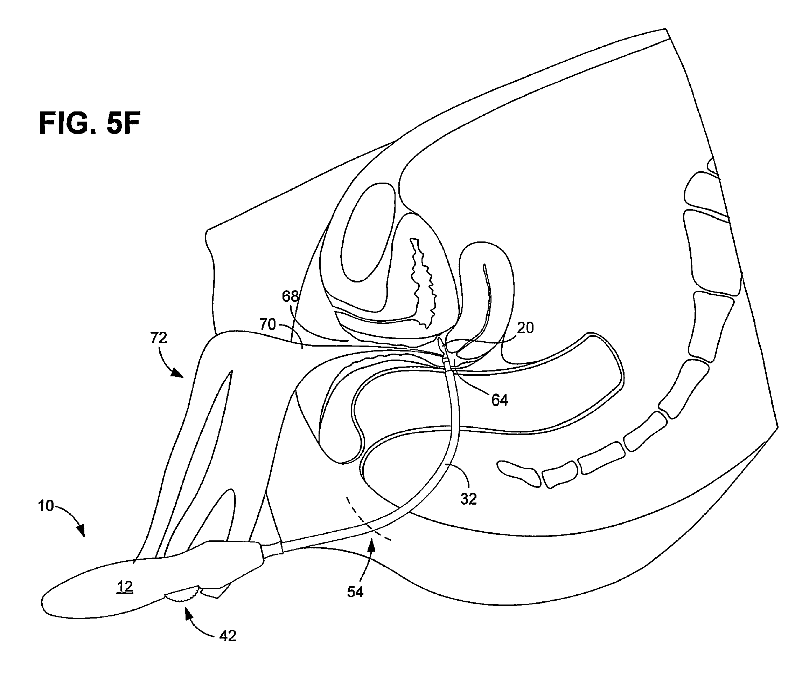

Turning to FIG. 5F, the snare 28 can then be retracted back into the introducer needle 18, for example using the mechanism 42, such that the implant coupling element 38 is again substantially contained within the inner lumen of the needle.

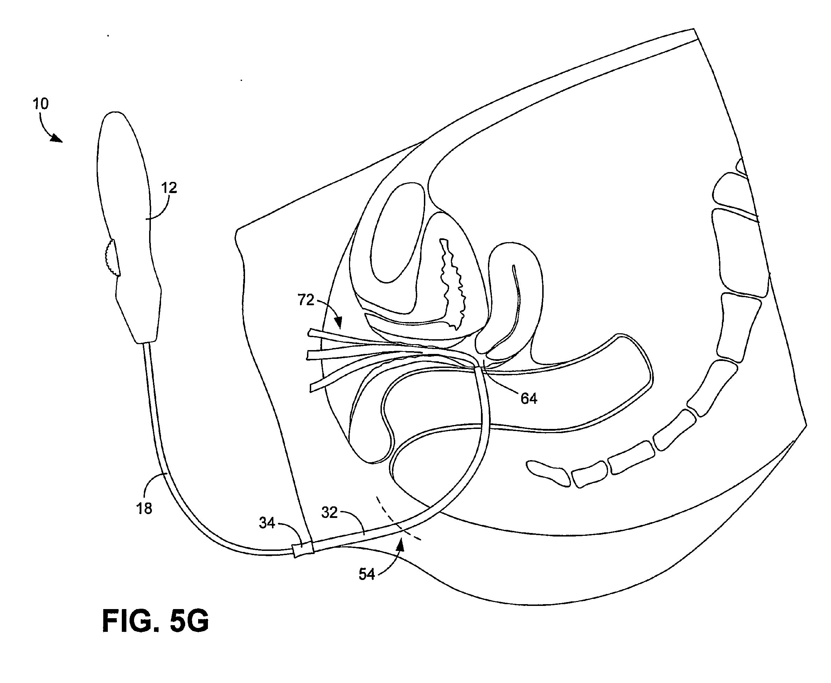

With reference next to FIG. 5G, needle 18 next can be withdrawn from the sheath 32 with the sheath maintained in place within the body so as to pull the implant arm 70 through the sheath and position the arm within the passage formed by the needle during insertion. This can be achieved by the surgeon holding the gripping element 34 with his or her hand to prevent movement of the sheath 32, and the surgeon withdrawing the needle 18 by pulling and upwardly rotating the handle 12 away from the body. When such a procedure is performed, the sheath 32 can be placed in the fully-extended position (FIG. 4C) such that the gripping element 34 abuts against the needle stop 36 (FIGs. 3A and 3B). It is noted that, because the implant arm 70 is positioned while enclosed by the sheath 32, unnecessary damage or irritation to the soft tissues in which the passage has been formed is reduced, as is the friction that resists such positioning.

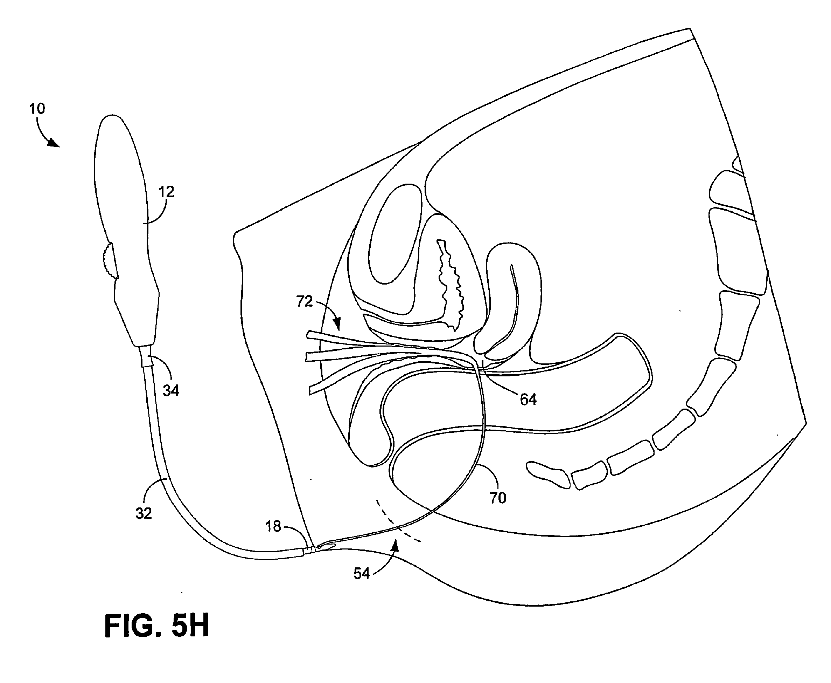

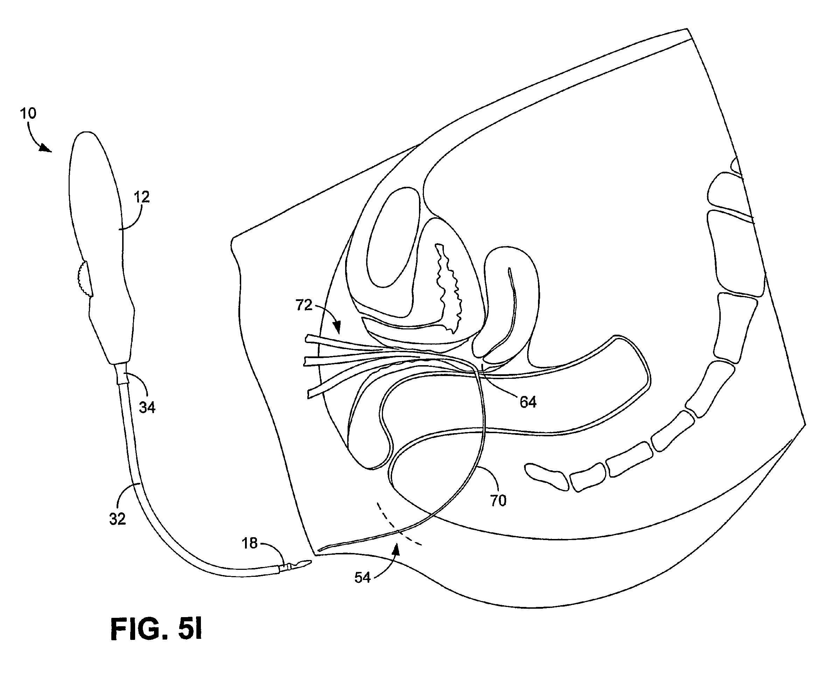

Next, as indicated in FIG. 5H, the sheath 32 can be drawn back over the needle 18 to return the sheath to the fully-retracted position (FIG. 4A). At that point, the tip 20 of the needle 18 is again exposed, thereby enabling the surgeon to release the implant arm 70 from the implant coupling element 28 so, as indicated in FIG. 5I, the implant can be detached from the introducer 10.

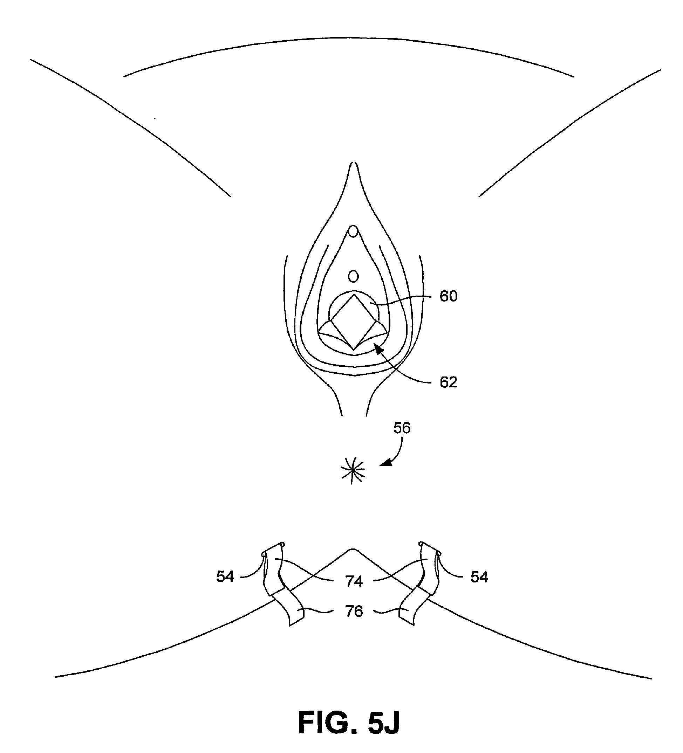

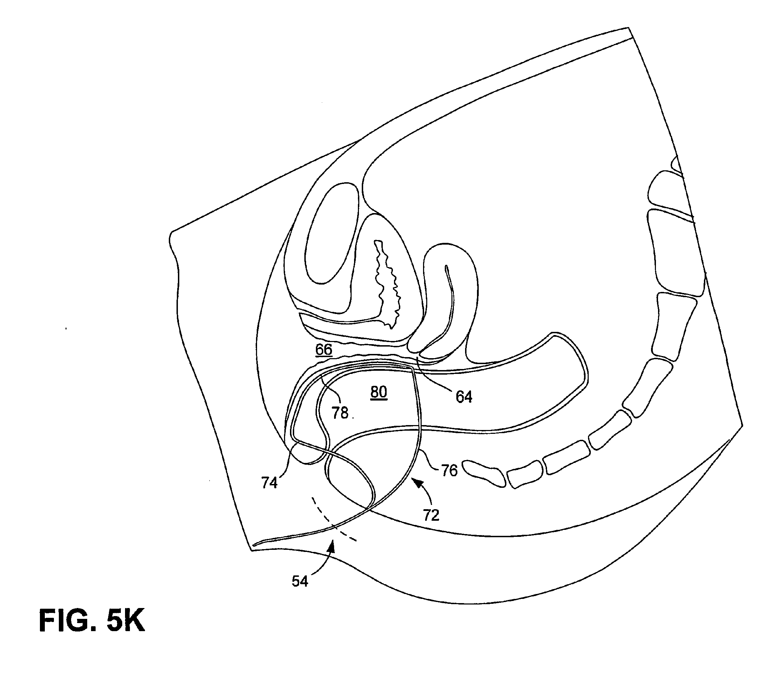

A similar procedure can then be followed for positioning the opposite arm of the implant 72 using the other pararectal incision 54. That is, the introducer needle 18 and its sheath 32 can be passed through the incision 54 to the vaginal vault 64 on the opposite side of the vagina 66 and the opposite implant arm can be positioned in the passage formed by the needle by drawing the arm through the sheath 32. In addition, relatively short arms of the implant 72 can be positioned in other passages extending from the incisions 54 on opposite sides of the vagina 66 to a position adjacent the vaginal introitus. Once that has been completed, a portion of a relatively short arm 74 and a portion of a relatively long arm 76 extends out from each pararectal incision 54, as indicated in FIG. 5J, and a central body 78 (FIG. 5K) of the implant 70 can be positioned between the vagina 66 and the rectum 80 to provide a support structure that prevents encroachment of the rectum into the vaginal space. The implant arms can then be appropriately tensioned, for example by pulling excess length out from the incisions 54, and the portions of the arms 74, 76 that extend outside of the body trimmed. Finally, the midline incision in the vaginal wall is closed. The final result of the implantation is illustrated in FIG. 5K, with the implant body 78 positioned between the vagina 66 and the rectum 80.

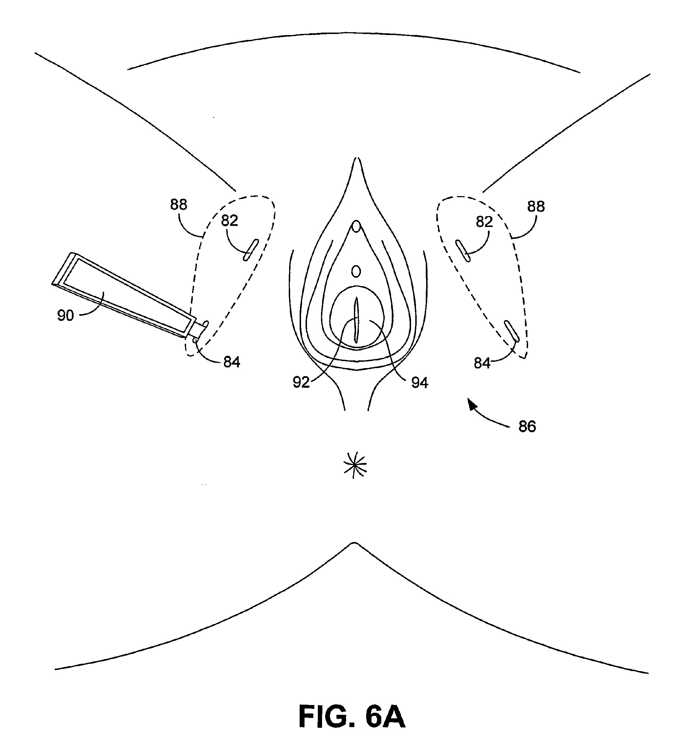

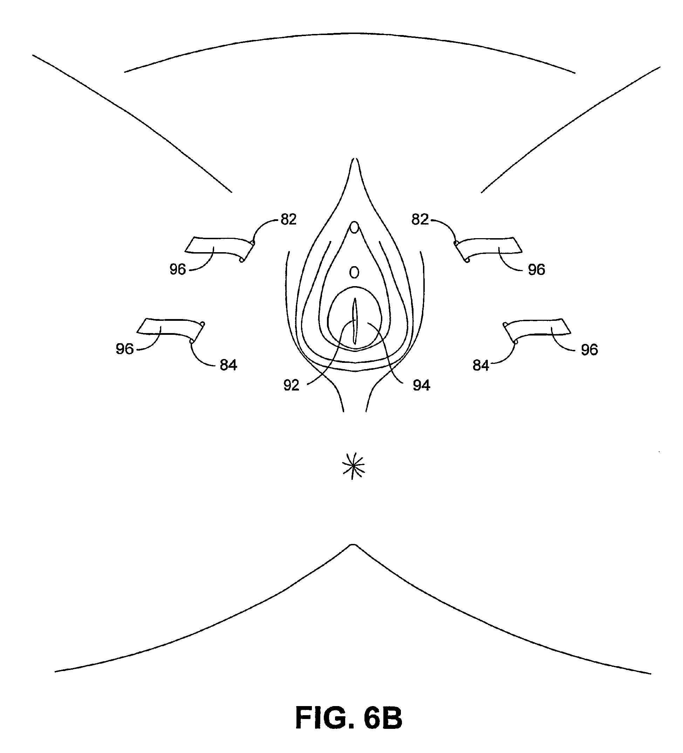

As described above, other implantation procedures can be performed using similar introducers. For example, anterior prolapse repair can be performed. To perform such a procedure, similar steps to those described above are completed. The primary differences include the shape of the implant, the location of the incisions made in the pelvis, and the positioning of the implant within the pelvis. As shown in FIG. 6A, superior and inferior incisions 82 and 84 can be made in the paravaginal region 86 in alignment with the obturator foramina 88 of the pubic bone. Again, those incisions 82 and 84 can be made with a sharp device, such as a scalpel 90. In addition, a midline incision 92 can be made in the anterior vaginal wall 94 to provide access to the space between the vagina and the urethra. Each of four arms of an implant can be positioned within passages that extend from the incisions 82 and 84 to the vagina to position a body of the implant between the vagina and the urethra. As shown in FIG. 6B, portions of the arms 96 that extend from the incisions 82 and 84 can be trimmed as described above in relation to the posterior prolapse repair procedure.

As is also described above, introducers in accordance with this disclosure can be used to treat urinary incontinence. In such a procedure, similar steps are performed except that the implant can comprise a urethral sling that is positioned below the urethra to provide support to the urethra. The ends of the sling can, for example, be passed through and/or embedded in the fibrous tissue of the obturator foramina, or can be otherwise secured to hard or soft tissue of the pelvis.

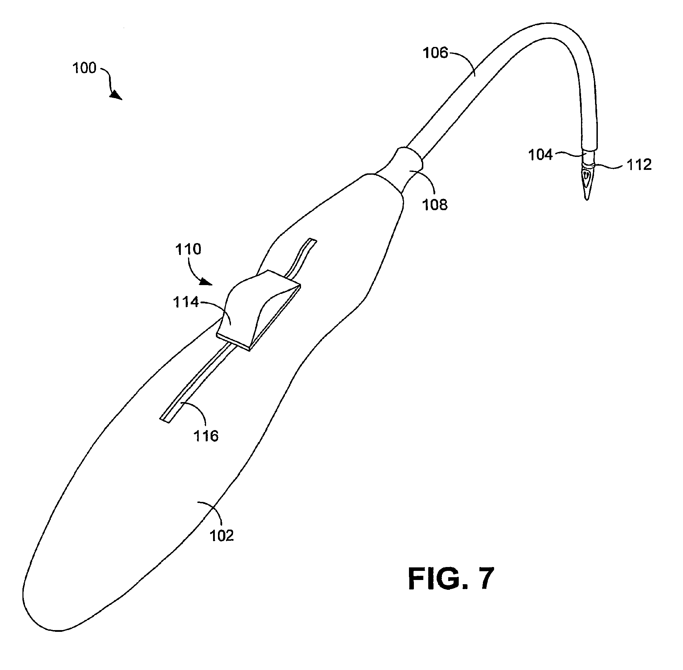

FIG. 7 illustrates an alternative embodiment of an introducer 100. As indicated in that figure, the introducer 100 comprises a handle 102 and a needle 104. As with the previously-described embodiment, provided on the needle 104 is an external sheath 106 that includes a gripping element 108. In addition, the introducer 100 includes a mechanism 110 for extending and retracting an internal snare 112 of the introducer. In the embodiment of FIG. 7, however, the mechanism 110 comprises a slide element 114 that can be moved along a slot 116 formed in the handle 102. During use of the introducer 100, movement of the slide element 114 in a first direction along the slot 116 causes extension of the snare 112, while movement of the slide element in an opposite direction along the slot causes retraction of the snare.

## Claims

1. An introducer for introducing an implant into a patient's body, comprising:
a needle (18) having an inner lumen and an opening (48) that provides access to the inner lumen;
an extendible snare (28) positioned within the inner lumen of the needle (18), the snare (28) having an implant coupling element (38), wherein the snare (28) is extendable from the needle opening (48) to an extended position in which the implant coupling element (38) is positioned outside of the inner lumen and retractable to a retracted position in which the implant coupling element (38) is positioned within the inner lumen; and
a sheath (32) provided on the needle (18), the sheath (32) being positionable in a first position in which the sheath (32) substantially surrounds the needle (18) and a second position in which the sheath extends beyond a distal tip (20) of the needle (18), **characterised in that** the distal tip (20) is a blunt tip (20), and **in that** the needle (18) comprises a depression (46) adjacent to its needle tip (20), in which depression (46) the opening (48) is provided.

2. The introducer of claim 1, wherein the needle (18) is elongated and curved.

3. The introducer of claim 1, further comprising a handle (12), wherein the needle (18) comprises a generally straight portion (22) adjacent the handle (12), a curved portion (24) in a central region, and a second generally straight portion (26) adjacent its tip (20).

4. The introducer of claim 1, wherein the implant coupling element (38) comprises a loop configured to receive a portion of an implant.

5. The introducer of claim 4, wherein the loop is constructed of nitinol.

6. The introducer of claim 4, wherein the loop includes a constriction configured to securely clamp the implant.

7. The introducer of claim 1, further comprising a handle (12) to which the needle is attached.

8. The introducer of claim 7, wherein the handle (12) comprises a mechanism that is configured to extend and retract the snare.

9. The introducer of claim 8, wherein the mechanism comprises a rotatable element.

10. The introducer of claim 8, wherein the mechanism comprises a slide element.

11. The introducer of claim 1, wherein the sheath (32) is made of a flexible polymeric material.

12. The introducer of claim 1, wherein the tip (20) of the needle (18) is exposed when the sheath (32) is in the first position.

13. The introducer of claim 1, wherein the sheath (32) comprises a gripping element that enables the needle (18) to be withdrawn from a patient while maintaining the sheath (32) in place within the patient.

14. The introducer of claim 1, wherein the needle (18) comprises a stop (36) that limits extension of the sheath (32) relative to the needle (18) such that the sheath (32) cannot be completely removed from the needle (18).

15. The introducer of claim 14, wherein
the stop (36) is provided adjacent the distal tip (20).

## Patentansprüche

1. Einführvorrichtung zum Einführen eines Implantats in den Körper eines Patienten, aufweisend:
eine Nadel (18), die ein inneres Lumen und eine Öffnung (48) aufweist, die einen Zugang zu dem inneren Lumen bildet;
eine ausziehbare Schlinge (28), die in dem inneren Lumen der Nadel (28) positioniert ist, wobei die Schlinge (28) ein Implantatkupplungselement (38) aufweist, wobei die Schlinge (28) aus der Nadelöffnung (48) in eine Ausziehposition herausziehbar ist, in welcher das Implantatkupplungselement (38) außerhalb des inneren Lumens positioniert ist, und in eine Rückziehposition zurückziehbar ist, in welcher das Implantatkupplungselement (38) in dem inneren Lumen positioniert ist; und
eine Hülle (32), die an der Nadel (18) vorgesehen ist, wobei die Hülle (32) in einer ersten Position, in welcher die Hülle (32) die Nadel (18) im Wesentlichen umschließt, und einer zweiten Position positionierbar ist, in welcher sich die Hülle über eine distale Spitze (20) der Nadel (18) hinaus erstreckt, **dadurch gekennzeichnet,**
**dass** die distale Spitze (20) eine stumpfe Spitze (20) ist, und
**dass** die Nadel (18) benachbart zu ihrer Nadelspitze (20) eine Vertiefung (46) aufweist, in welcher Vertiefung (46) die Öffnung (48) vorgesehen ist.

2. Einführvorrichtung nach Anspruch 1, wobei die Nadel (18) länglich und gekrümmt ist.

3. Einführvorrichtung nach Anspruch 1, ferner aufweisend einen Handgriff (12), wobei die Nadel (18) benachbart zu dem Handgriff (12) einen im Allgemeinen geraden Abschnitt (22), in einem mittleren Bereich einen gekrümmten Abschnitt (24), und benachbart zu ihrer Spitze (20) einen zweiten im Allgemeinen geraden Abschnitt (26) aufweist.

4. Einführvorrichtung nach Anspruch 1, wobei das Implantatkupplungselement (38) eine Öse aufweist, die derart konfiguriert ist, dass sie einen Abschnitt eines Implantats aufnimmt.

5. Einführvorrichtung nach Anspruch 4, wobei die Öse aus Nitinol konstruiert ist.

6. Einführvorrichtung nach Anspruch 4, wobei die Öse eine Einschnürung aufweist, die derart konfiguriert ist, dass sie das Implantat sicher einklemmt.

7. Einführvorrichtung nach Anspruch 1, ferner aufweisend einen Handgriff (12), an welchem die Nadel angebracht ist.

8. Einführvorrichtung nach Anspruch 7, wobei der Handgriff (12) einen Mechanismus aufweist, der derart konfiguriert ist, dass er die Schlinge heraus- und zurückzieht.

9. Einführvorrichtung nach Anspruch 8, wobei der Mechanismus ein drehbares Element aufweist.

10. Einführvorrichtung nach Anspruch 8, wobei der Mechanismus ein Gleitelement aufweist.

11. Einführvorrichtung nach Anspruch 1, wobei die Hülle (32) aus einem flexiblen Polymermaterial hergestellt ist.

12. Einführvorrichtung nach Anspruch 1, wobei die Spitze (20) der Nadel (18) freigelegt ist, wenn die Hülle (32) in der ersten Position ist.

13. Einführvorrichtung nach Anspruch 1, wobei die Hülle (32) ein Greifelement aufweist, das ermöglicht, dass die Nadel (18) aus einem Patienten herausgezogen werden kann, während die Hülle (32) an Ort und Stelle in dem Patienten belassen werden kann.

14. Einführvorrichtung nach Anspruch 1, wobei die Nadel (18) einen Anschlag (36) aufweist, der die Erstreckung der Hülle (32) relativ zu der Nadel (18) derart begrenzt, dass die Hülle (32) nicht vollständig von der Nadel (18) entfernt werden kann.

15. Einführvorrichtung nach Anspruch 14, wobei der Anschlag (36) benachbart zu der distalen Spitze (20) vorgesehen ist.

## Revendications

1. Dispositif d'introduction pour introduire un implant dans le corps d'un patient, comprenant :
une aiguille (18) ayant une lumière interne et une ouverture (48) qui fournit l'accès à la lumière interne ;
une anse extensible (28) positionnée à l'intérieur de la lumière interne de l'aiguille (18), l'anse (28) ayant un élément de couplage d'implant (38), dans lequel l'anse (28) est extensible de l'ouverture d'aiguille (48) à une position étendue dans laquelle l'élément de couplage d'implant (38) est positionné à l'extérieur de la lumière interne et rétractable dans une position rétractée dans laquelle l'élément de couplage d'implant (38) est positionné à l'intérieur de la lumière interne ; et
une gaine (32) prévue sur l'aiguille (18), la gaine (32) pouvant être positionnée dans une première position dans laquelle la gaine (32) entoure sensiblement l'aiguille (18) et une deuxième position dans laquelle la gaine s'étend au-delà d'une pointe distale (20) de l'aiguille (18), **caractérisé en ce que** la pointe distale (20) est une pointe émoussée (20) et **en ce que** l'aiguille (18) comprend un enfoncement (46) adjacent à sa pointe d'aiguille (20), dans lequel enfoncement (46), l'ouverture (48) est prévue.

2. Dispositif d'introduction selon la revendication 1, dans lequel l'aiguille (18) est allongée et incurvée.

3. Dispositif d'introduction selon la revendication 1, comprenant en outre une poignée (12), dans lequel l'aiguille (18) comprend une partie (22) généralement droite adjacente à la poignée (12), une partie incurvée (24) dans une région centrale, et une deuxième partie (26) généralement droite adjacente à sa pointe (20).

4. Dispositif d'introduction selon la revendication 1, dans lequel l'élément de couplage d'implant (38) comprend une boucle configurée pour recevoir une partie d'un implant.

5. Dispositif d'introduction selon la revendication 4, dans lequel la boucle est construite à partir de Nitinol.

6. Dispositif d'introduction selon la revendication 4, dans lequel la boucle comprend une constriction configurée pour serrer fermement l'implant.

7. Dispositif d'introduction selon la revendication 1, comprenant en outre une poignée (12) à laquelle l'aiguille est fixée.

8. Dispositif d'introduction selon la revendication 7, dans lequel la poignée (12) comprend un mécanisme qui est configuré pour étendre et rétracter l'anse.

9. Dispositif d'introduction selon la revendication 8, dans lequel le mécanisme comprend un élément rotatif.

10. Dispositif d'introduction selon la revendication 8, dans lequel le mécanisme comprend un élément coulissant.

11. Dispositif d'introduction selon la revendication 1, dans lequel la gaine (32) est réalisée à partir d'un matériau polymère souple.

12. Dispositif d'introduction selon la revendication 1, dans lequel la pointe (20) de l'aiguille (18) est exposée lorsque la gaine (32) est dans la première position.

13. Dispositif d'introduction selon la revendication 1, dans la gaine (32) comprend un élément de préhension qui permet à l'aiguille (18) d'être retirée d'un patient tout en maintenant la gaine (32) en place dans le patient.

14. Dispositif d'introduction selon la revendication 1, dans lequel l'aiguille (18) comprend une butée (36) qui limite l'extension de la gaine (32) par rapport à l'aiguille (18) de sorte que la gaine (32) ne peut pas être complètement retirée de l'aiguille (18).

15. Dispositif d'introduction selon la revendication 14, dans lequel la butée (36) est prévue de manière adjacente à la pointe distale (20).
